(19) Europäisches Patentamt
European Patent Office
Office européen des brevets

(11) EP 0 854 128 B1

(12) EUROPEAN PATENT SPECIFICATION

(45) Date of publication and mention of the grant of the patent:
**25.07.2001 Bulletin 2001/30**

(51) Int Cl.[7]: **C07C 51/58**, C07C 63/70, C07C 45/46, C07C 49/813, C07C 49/84

(21) Application number: **98300210.6**

(22) Date of filing: **13.01.1998**

(54) **Process for the preparation of chloro-benzoyl chlorides**

Verfahren zur Herstellung von Chlor-benzoylchlorid

Procédé pour la préparation du chlorure de chlorobenzoyle

(84) Designated Contracting States:
**AT BE CH DE DK ES FI FR GB GR IE IT LI LU NL PT SE**

(30) Priority: **15.01.1997 EP 97100518**

(43) Date of publication of application:
**22.07.1998 Bulletin 1998/30**

(73) Proprietor: **BASF AKTIENGESELLSCHAFT**
**67056 Ludwigshafen (DE)**

(72) Inventors:
- **Van den Akker, Laurens Wilhem**
  **678 EX De Lier (NL)**
- **Brink, Monika**
  **55218 Ingelheim (DE)**

(74) Representative: **Langfinger, Klaus Dieter, Dr. et al**
**Director Patents & Licensing,**
**BASF Aktiengesellschaft ZDX/P-C6**
**67056 Ludwigshafen (DE)**

(56) References cited:
**EP-A- 0 273 528** **US-A- 1 878 463**
**US-A- 3 230 268**

- **DATABASE WPI Section Ch, Week 8049 Derwent Publications Ltd., London, GB; Class A41, AN 80-87950C XP002059967 -& SU 729 186 A (USPENSKAYA I N) , 28 April 1980**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 854 128 B1

Printed by Jouve, 75001 PARIS (FR)

## Description

The present invention relates to a process for the preparation of chloro-benzoylchlorides from a chloro-trichloromethylbenzene. Chloro-trichloromethylbenzene compounds are suitable intermediates for the preparation of a broad variety of compounds which are useful as agrochemicals, pharmaceuticals or liquid crystals. In particular, they are key intermediates in the preparation of fungicidal dimethomorph (e.g. EP 0 120 321) and fungicidal benzophenones which are described for example in EP 0 727 141 A.

The German patent no. 331696 (July 17, 1914) discloses a process wherein trichloromethylbenzene is treated with equimolar amounts of water in the presence of $FeCl_3$ at elevated temperatures to form benzoylchloride. However, this process is difficult to carry out on a technical scale in view of problems with respect to the dosing rate and the exact equimolar dosing of water at elevated temperatures. Any excess of water will cause hydrolysis of the desired acid chloride compound and therefore reduce the yields.

The present invention provides an effective and efficient process for the preparation of chloro-benzoylchlorides of formula I,

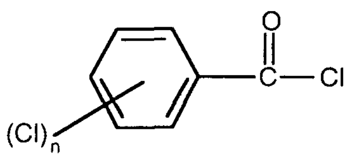

(I)

wherein
n represents an integer 1, 2 or 3,
from a chloro-trichloromethylbenzene of formula II

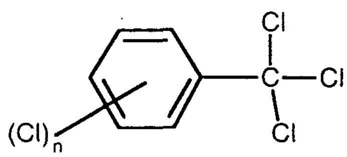

(II)

wherein n is as defined above,
characterised in that the trichloromethylarene is treated with water in the presence of a Lewis acid at temperatures below 80 °C.
It is, therefore, an object of the present invention to provide an efficient new process for the preparation of chloro-benzoylchloride compounds.
Other objects and advantages of the present invention will be apparent to those skilled in the art from the following description and the appended claims.

Further preferred embodiments of the process according to the present invention is a process wherein:

- the reaction is carried out without organic solvent;
- the Lewis acid is $FeCl_3$,
- the reaction mixture essentially consisting of the chloro-trichloromethylbenzene of formula II and the Lewis acid is heated up to temperatures below 80 °C and water is slowly added into the stirred solution;
- the chloro-trichloromethylbenzene is a mono- or dichlorotrichloromethylbenzene, in particular 4-chloro-trichloromethylbenzene or 2,6-dichlorotrichloromethylbenzene;
- I mole of trichloromethylarene of formula II is treated with 0.8 to 1.2, preferably 0.9 to 1.1, in particular about 1 moles of water;
- 1 mole of trichloromethylarene of formula II is treated with water in the presence of 0.05 to 5 mol-%, preferably 0.1 to 3 mol-% of the Lewis acid.

The reaction is carried out at a temperature between ambient temperature and 80 °C, preferably between 40 and 80 °C, in particular between 45 and 65 °C.

As a rule the reaction can be carried out under reduced or elevated pressure, preferably it is carried out under ambient pressure.

In a particularly preferred embodiment of the process according to the invention 1 equivalent of 4-chloro-trichloromethylbenzene, is mixed with 0.1 to 3 mol-% of the Lewis acid, in particular $FeCl_3$ and heated to 45 to 65°C, in particular 50 to 60 °C. Then about 1 equivalent of water is slowly dosed (eg over a period of 1 to 2 hours or more) into the stirred reaction mixture, which is kept at this temperature. The water may be introduced via one or more inlets, preferably via one or two inlets at a dosing rate of 0.1 to 0.8 ml/min, preferably 0.2 to 0.5 ml/min. In another preferred embodiment the water is dosed as steam instead as liquid. As a rule, water is lead in a defined rate through a pipe which is heated to 140 °C using an inert atmosphere, preferably nitrogen or argon as steam carrier. The steam preferably is introduced into the reaction mixture in the form of a fine dispersion of the reagents. In order to prevent the formation of benzoic anhydrides as a by-product the steam is introduced using less than 50 %, preferably about 25 % nitrogen as carrier. Preferably the mixing of the steam with the reaction mixture is enhanced by one or more baffles.
The hydrochloric acid formed during the reaction is evolved out until the reaction is completed.
Under these preferred reaction conditions the reaction is as a rule completed within 0.25 to 8, in particular 4 to 6 hours.
It has been found that the reaction time depends on the stirring rate of the reaction mixture. Optimal reaction conditions were found with a stirring power input of 0.5 to 2.0 kW/m3, in particular of about 1.0 kW/m3.
The dependence of the stirring power input and the volume of the reaction mixture, the stirrer geometry and the stirring rate is given by the following formula:

$$P = P_0 * \rho * n^3 * d/V$$

P = power input
$P_0$ = standard value for a certain stirrer
$\rho$ = density
n = stirring speed
d = diameter of the stirrer
V = volume

The remaining benzoylchloride can be used as intermediate for the preparation of the desired end-products without further purification. In a particularly preferred embodiment the reaction mixture obtained can be used for the preparation of certain chloro-substituted benzophenone derivatives under Friedel-Crafts acylation conditions, in particular of formula III,

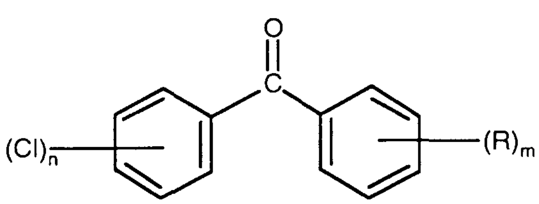

(III)

in which n has the meaning given,
R each independently represents $C_{1-6}$ alkyl or $C_{1-6}$ alkoxy, and
m is an integer from 2 to 4,
under Friedel-Crafts acylation conditions by adding the corresponding substituted benzene derivative, preferably of formula IV,

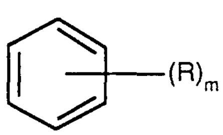

(IV)

in particular 1,2-dimethoxybenzene, 3,4,5-trimethoxytoluene or 3,4-dimethoxy-5-butoxytoluene, and optionally adding a Lewis acid to said reaction mixture, preferably without adding additional Lewis acid.
It is also possible to purify the obtained chloro-benzoylchlorides using standard procedures, as for example

crystallization or distillation, in particular by distillation under reduced pressure, in particular at pressures between I and 100 mbar.

The novel process enables to carry out the production of chloro-benzoyl chlorides in technical scale and high yields using cheap, readily-available materials. In order to facilitate a further understanding of the invention, the following illustrative examples are presented. The invention is not limited to the specific embodiments described or illustrated, but encompasses the full scope of the appended claims.

**Example 1**

Preparation of 4-chlorobenzoylchloride (PCBO)

A mixture of 115 g 4-chlorobenzotrichloride (CBTC, 0.5 mol) and 1.6 g $FeCl_3$ (10 mmol) is heated to 60°C. 9 g water (0.5 mol) are dosed into the mixture within 1 hours at ambient pressure. After 30 minutes the reaction was complete; the GC analysis showed that the conversion was complete (98 area-% PCBO). In order to determine the yield, the corresponding methyl-ester has been made by adding 1,5 equivalent methanol to the obtained product. The resulting mixture was disolved in toluene and washed with 5% aqueous HCl and water. After removing the solvent and recrystallization 83,3 g p-chloromethylbenzoate (98% purity) was formed. Total yield 93%.

**Example 2**

Preparation of 4-chlorobenzoylchloride (PCBO)

A mixture of 460 g 4-chlorobenzotrichloride (CBTC, 2 mol) and 6.5 g $FeCl_3$ (20 mmol) is heated to 50°C. 36 g water (2 mole) are dosed into the mixture within 2 hours at ambient pressure. After 30 minutes the reaction was complete; the GC analysis showed that the conversion was complete ( >95 area-% PCBO).

The results of further experiments are given in the following table, in which the conversions have been determined by GC analysis:

Table 1 :

| GC-area% PCBO of mixture | | | |
|---|---|---|---|
| **CBTC (mol)** | **$FeCl_3$ (mol %)** | **T (°C)** | **GC-area % PCBO** |
| 1,5 | 2 | 53 | 97 |
| 2 | 2 | 51 | 97 |
| 2 | 2 | 60 | 98 |

In the following experiments the yield of PCBO has been determined as the actual content of the product given in weight % as shown in Table 2:

Table 2:

| Weight% PCBO of mixture | | | | | |
|---|---|---|---|---|---|
| **CBTC (mol)** | **$FeCl_3$ (mol %)** | **Stirrer (rpm)** | **Dosing time (h)** | **T (°C)** | **weight % PCBO** |
| 2 | 0.5 | 420 | 4 | 50 | 94.2 |
| 2 | 0.25 | 420 | 4 | 50 | 95.3 |
| 3.25 | 0.4 | 388 | 6 | 50 | 95.5 |
| 3.25 | 0.25 | 388 | 4 | 70 | 88.6 |
| 3.25 | 0.25 | 388 | 4 | 50 | 96.3 |

**Example 3**

Preparation of 4-chlorobenzoylchloride (PCBO)

A mixture of 3736 g 4-chlorobenzotrichloride (CBTC, 16.25 mol) and 6,6 g $FeCl_3$ (41 mmol) is heated to 50°C.

292 g water (16.25 mol) as steam mixed with 25 % nitrogen as carrier dosed are into the mixture within 8 hours at ambient pressure. After 30 minutes post-reaction time the reaction was complete; the GC analysis showed that the conversion was complete (97.4 area-% PCBO).

**Use Example**

Preparation of 4-chloro-3',4'-dimethoxybenzophenone (CDMBP)

552 g 1,2-dimethoxybenzene (4.0 mol) are added to a reaction mixture containing 703 g 4-chlorobenzoylchloride (PCBO, 4.0 mol) and $FeCl_3$ (1 mmol) as obtained from example 3. The reaction mixture is heated to 135 °C for 30 minutes. After usual work-up 1030 g of CDMBP (93 %) are isolated as pure product, melting point 109-111 °C.

**Comparison Example**

Preparation of 4-chloro-benzoylchloride

A mixture of 460 g 4-chlorobenzotrichloride (CBTC, 2 mol) and 1.0 g $FeCl_3$ («20/6,5 » mmol) is heated to 100°C. 36 g water (2 mol) are dosed into the mixture within 2 hours at ambient pressure. After 30 minutes the analysis showed that the conversion was incomplete (85 weight % PCBO).

## Claims

1. A process for the preparation of a chloro-benzoylchloride of formula I,

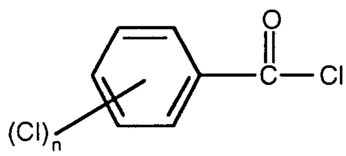

(I)

wherein
n represents an integer 1, 2 or 3,
from a chloro-trichloromethylbenzene of formula II

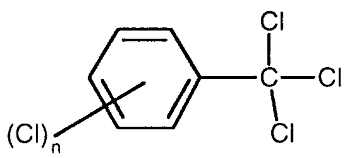

(II)

wherein n is as defined above,
characterised in that the trichloromethylarene is treated with water in the presence of a Lewis acid at temperatures at or below 80 °C.

2. A process according to claim 1, wherein 1 mole of chloro-trichloromethylbenzene of formula II is treated with 0.8 to 1.2 moles of water.

3. A process according to claim 1 or claim 2, wherein 1 mole of trichloromethylarene of formula II is treated with water in the presence of 0.05 to 5.0 mol-% of the Lewis acid.

4. A process as claimed in any one of claims 1 to 3 when carried out at a temperature of 40 to 80°C.

5. A process as claimed in any one of claims 1 to 3 when carried out at a temperature from 45 to 65°C.

**6.** A process according to any of the preceding claims, wherein about 1 equivalent of water is slowly dosed into a mixture essentially consisting of 1 equivalent of chloro-trichloromethylbenzene of formula II and 0.05 to 3.0 mol-% of the Lewis acid at 40 to 80°C.

**7.** A process according to any of the preceding claims, wherein the water is introduced via one or more inlets at a dosing rate of 0.1 to 0.8 ml/min.

**8.** A process according to any of the preceding claims, wherein the water is dosed as steam using an inert-gas as steam carrier.

**9.** A process according to any of the preceding claims, wherein the reaction mixture is stirred during the introduction of water with a stirring power input of 0.5 to 2.0 kW/m3.

**10.** A process according to any one of the preceding claims, wherein the Lewis acid is $FeCl_3$.

**11.** A process according to any one of the preceding claims in which the chloro-benzoylchlorides of formula I prepared is further reacted under Friedel-Crafts acylation conditions with a substituted benzene derivative of formula IV,

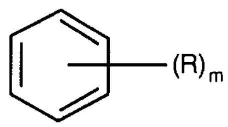

(IV)

wherein each R independently represents $C_{1-6}$ alkyl or $C_{1-6}$ alkoxy, and
m is an integer from 2 to 4,
and optionally a Lewis acid to give a chloro-substituted benzophenone derivative of formula III,

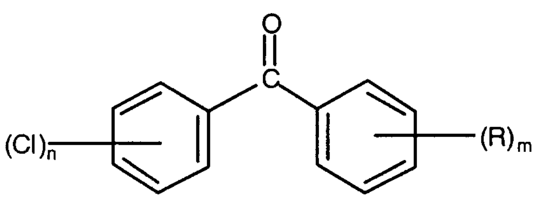

(III)

in which n is an integer 1, 2 or 3, and R and m are as defined above.

**12.** A process according t o any one of the preceding claims in which the reaction mixture containing the chloro-benzoylchlorides of formula I is further reacted under Friedel-Crafts acylation conditions with a substituted benzene derivative of formula IV,

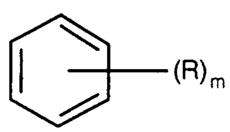

(IV)

wherein each R independently represents $C_{1-6}$ alkyl or $C_{1-6}$ alkoxy, and
m is an integer from 2 to 4,
and optionally a Lewis acid to give a chloro-substituted benzophenone derivative of formula III,

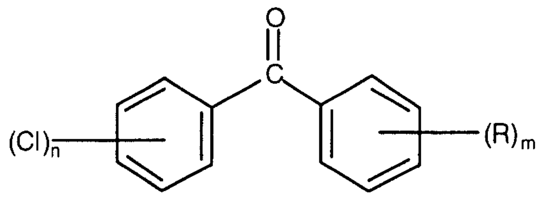

(III)

in which n is an integer 1, 2 or 3, and R and m are as defined above.

**Patentansprüche**

1. Verfahren zur Herstellung eines Chlorbenzoylchlorids der Formel I

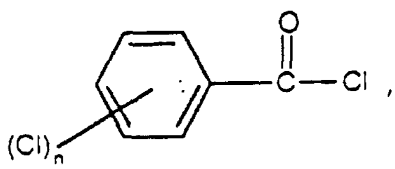

worin
n für die ganze Zahl 1, 2 oder 3 steht,
aus einem Chlor(trichlormethyl)benzol der Formel II

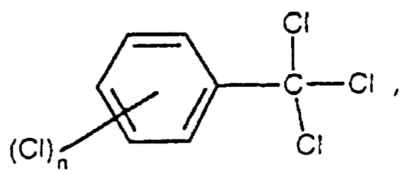

worin n die oben aufgeführte Bedeutung besitzt,
dadurch gekennzeichnet, daß man das (Trichlormethyl)aren in Gegenwart einer Lewis-Säure bei Temperaturen kleiner gleich 80°C mit Wasser behandelt.

2. Verfahren nach Anspruch 1, bei dem man 1 mol Chlor(trichlormethyl)benzol der Formel II mit 0,8 bis 1,2 mol Wasser behandelt.

3. Verfahren nach Anspruch 1 oder 2, bei dem man 1 mol (Trichlormethyl)aren der Formel II in Gegenwart von 0,05 bis 5,0 Mol-% der Lewis-Säure mit Wasser behandelt.

4. Verfahren nach einem der Ansprüche 1 bis 3, das man bei einer Temperatur von 40 bis 80°C durchführt.

5. Verfahren nach einem der Ansprüche 1 bis 3, das man bei einer Temperatur von 45 bis 65°C durchführt.

6. Verfahren nach einem der vorhergehenden Ansprüche, bei dem man eine im wesentlichen aus 1 Äquivalent Chlor (trichlormethyl)benzol der Formel II und 0,05 bis 3,0 Mol-% der Lewis-Säure bestehende Mischung bei 40 bis 80°C langsam mit etwa 1 Äquivalent Wasser versetzt.

7. Verfahren nach einem der vorhergehenden Ansprüche, bei dem man das Wasser über einen oder mehrere Zuläufe mit einer Zudosierungsrate von 0,1 bis 0,8 ml/min zugibt.

**8.** Verfahren nach einem der vorhergehenden Ansprüche, bei dem man das Wasser unter Verwendung eines Inertgases als Dampfträger als Dampf zudosiert.

**9.** Verfahren nach einem der vorhergehenden Ansprüche, bei dem man die Reaktionsmischung bei der Zugabe des Wassers mit einem Rührleistungseintrag von 0,5 bis 2,0 $kW/m^3$ rührt.

**10.** Verfahren nach einem der vorhergehenden Ansprüche, bei dem man als Lewis-Säure $FeCl_3$ einsetzt.

**11.** Verfahren nach einem der vorhergehenden Ansprüche, bei dem man die hergestellten Chlorbenzoylchloride der Formel I unter Friedel-Crafts-Acylierungsbedingungen mit einem substituierten Benzolderivat der Formel IV

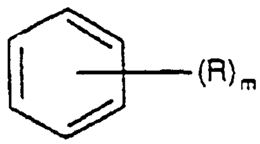

(IV) ,

worin R jeweils unabhängig für $C_{1-6}$-Alkyl oder $C_{1-6}$-Alkoxy und
m für eine ganze Zahl von 2 bis 4 steht,
und gegebenenfalls einer Lewis-Säure weiter zu einem chlorsubstituierten Benzophenon-Derivat der Formel III

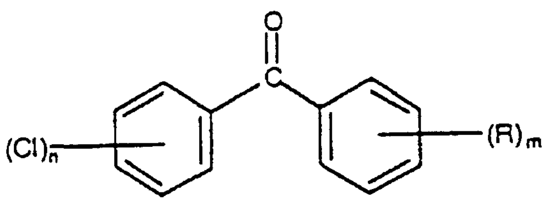

(III) ,

worin n für die ganze Zahl 1, 2 oder 3 steht und R und m die oben angegebenen Bedeutungen besitzen, umsetzt.

**12.** Verfahren nach einem der vorhergehenden Ansprüche, bei dem man die die Chlorbenzyolchloride der Formel I enthaltende Reaktionsmischung unter Friedel-Crafts-Acylierungsbedingungen mit einem substituierten Benzolderivat der Formel IV

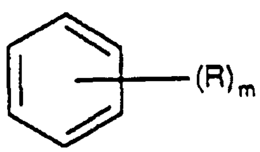

(IV) ,

worin R jeweils unabhängig für $C_{1-6}$-Alkyl oder $C_{1-6}$-Alkoxy und
m für eine ganze Zahl von 2 bis 4 steht,
und gegebenenfalls einer Lewis-Säure weiter zu einem chlorsubstituierten Benzophenon-Derivat der Formel III

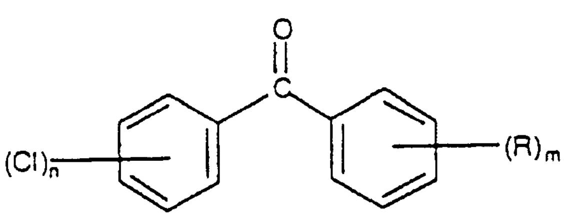

(III) ,

worin n für die ganze Zahl 1, 2 oder 3 steht und R und m die oben angegebenen Bedeutungen besitzen, umsetzt.

## Revendications

**1.** Procédé pour la préparation d'un chlorure de chlorobenzoyle de formule I,

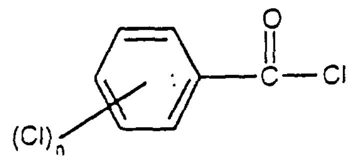

(I)

où n représente un nombre entier 1, 2 ou 3,
à partir d'un chloro-trichlorométhylbenzène de formule II

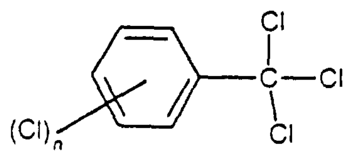

(II)

où n est tel que défini ci-dessus,
caractérisé en ce que le trichlorométhylarène est traité avec de l'eau en présence d'un acide de Lewis à des températures égales ou inférieures à 80°C.

**2.** Procédé selon la revendication 1, dans lequel 1 mole du chloro-trichlorométhylbenzène de formule II est traitée avec 0,8 à 1,2 mole d'eau.

**3.** Procédé selon la revendication 1 ou la revendication 2, dans lequel 1 mole du trichlorométhylarène de formule II est traitée avec de l'eau en présence de 0,05 à 5,0 mol % de l'acide de Lewis.

**4.** Procédé selon l'une quelconque des revendications 1 à 3, qui est exécuté une température de 40 à 80°C.

**5.** Procédé selon l'une quelconque des revendications 1 à 3, qui est exécuté à une température de 45 à 65°C.

**6.** Procédé selon l'une quelconque des revendications précédentes, dans lequel on introduit lentement environ 1 équivalent d'eau dans un mélange essentiellement constitué de 1 équivalent du chloro-trichlorométhylbenzène de formule II et 0,05 à 3,0 mol % de l'acide de Lewis entre 40 et 80°C.

**7.** Procédé selon l'une quelconque des revendications précédentes, dans lequel l'eau est introduite par une ou plusieurs entrées à un débit réglé de 0,1 à 0,8 ml/min.

**8.** Procédé selon l'une quelconque des revendications précédentes, dans lequel l'eau est introduite à débit réglé sous forme de vapeur d'eau en utilisant un gaz inerte comme gaz porteur de vapeur d'eau.

**9.** Procédé selon l'une quelconque des revendications précédentes, dans lequel, pendant l'introduction de l'eau, le mélange réactionnel est agité avec une puissance fournie d'agitation de 0,5 à 2,0 $kW/m^3$.

**10.** Procédé selon l'une quelconque des revendications précédentes, dans lequel l'acide de Lewis est $FeCl_3$.

**11.** Procédé selon l'une quelconque des revendications précédentes, dans lequel le chlorure de chlorobenzoyle de formule I préparé est amené à réagir encore dans des conditions d'acylation de Friedel-Crafts avec un dérivé substitué du benzène de formule IV

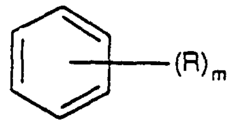

(IV)

où chaque R représente indépendamment un groupe alkyle en $C_1$-$C_6$ ou alcoxy en $C_1$-$C_6$, et m est un nombre entier de 2 à 4,
et facultativement un acide de Lewis pour donner un dérivé de benzophénone substitué par du chlore de formule III

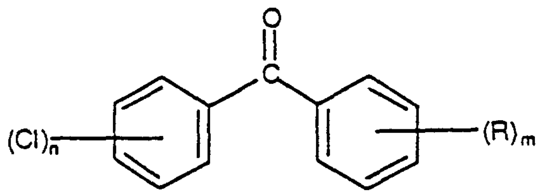

(III)

où n est un nombre entier 1, 2 ou 3 et R et m sont tels que définis ci-dessus.

**12.** Procédé selon l'une quelconque des revendications précédentes, dans lequel le mélange réactionnel contenant les chlorures de chlorobenzoyle de formule I est amené à réagir encore dans des conditions d'acylation de Friedel-Crafts avec un dérivé substitué du benzène de formule IV

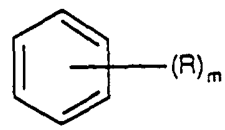

(IV)

où chaque R représente indépendamment un groupe alkyle en $C_1$-$C_6$ ou alcoxy en $C_1$-$C_6$, et m est un nombre entier de 2 à 4,
et facultativement un acide de Lewis pour donner un dérivé de benzophénone substitué par du chlore de formule III

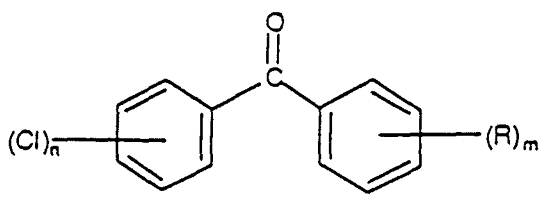

(III)

où n est un nombre entier 1, 2 ou 3 et R et m sont tels que définis ci-dessus.